# EUROPÄISCHE PATENTSCHRIFT

(11) **EP 1 015 888 B1**
(45) Veröffentlichungstag und Bekanntmachung des Hinweises auf die Patenterteilung: **28.08.2002**
(21) Anmeldenummer: 98948990.1
(22) Anmeldetag: 18.09.1998
(51) Int. Cl.: G01N 33/68, G01N 33/569

(54) **VERFAHREN ZUR MESSUNG DER ASSOZIATION VON TEILSTRUKTUREN PATHOLOGISCHER PROTEINABLAGERUNGEN**
METHOD FOR MEASURING THE ASSOCIATION OF SUBSTRUCTURES OF PATHOLOGICAL PROTEIN DEPOSITS
PROCEDE POUR MESURER L'ASSOCIATION DES STRUCTURES PARTICULAIRES DE DEPOTS PROTEIQUES PATHOLOGIQUES

(30) Priorität: 19.09.1997 DE 19741486; 28.11.1997 DE 19752712; 28.04.1998 DE 19818917
(43) Veröffentlichungstag der Anmeldung: 05.07.2000
(73) Patentinhaber: Evotec OAI AG, 22525 Hamburg (DE)
(72) Erfinder: RIESNER, Detlev, D-41541 Dormagen (DE); POST, Karin, D-40223 Düsseldorf (DE); SCHÄFER, Oliver, D-45239 Essen (DE); PITSCHKE, Martin, D-42553 Velbert (DE); EIGEN, Manfred, D-37075 Göttingen (DE); BIESCHKE, Jan, D-37081 Göttingen (DE)
(74) Vertreter: Meyers, Hans-Wilhelm, Dr.Dipl.-Chem.
(86) Internationale Anmeldenummer: EP9805958
(87) Internationale Veröffentlichungsnummer: WO99015903

(56) Entgegenhaltungen:
- WO-A-93/23432
- WO-A-97/10505
- WO-A-97/15685

## Beschreibung

Die vorliegende Erfindung betrifft ein Verfahren zur diagnostischen Erfassung von mit pathologischen Proteinablagerungen verbundenen Erkrankungen.

Eine Reihe verschiedener Erkrankungen ist mit dem Auftreten von pathologischen Proteinablagerungen verbunden. Dabei ist oft unklar, ob die Proteinablagerungen nur Erscheinungen eines Krankheitsbildes sind oder ob diese Proteinablagerungen kausal für die Erkrankung selbst als Pathogene verantwortlich sind. So sind neurodegenerative Erkrankungen bekannt, bei denen beispielsweise im Gehirn der Betroffenen als amyloide Plaques bezeichnete Proteinablagerungen feststellbar sind. Zu solchen Erkrankungen gehören beispielsweise die Alzheimersche Erkrankung, bovine spongiforme Enzephalopathie (BSE), die Creutzfeldt-Jakob-Erkrankung (CJD), Kuru-Erkrankung, Scrapie sowie möglicherweise auch andere Erkrankungen, die in der Vergangenheit auch als "slow-virus"-Erkrankungen bezeichnet wurden. In jüngster Zeit ist insbesondere die BSE-Erkrankung ins Bewußtsein der Öffentlichkeit gerückt, unter anderem, weil BSE mit der Creutzfeldt-Jakob-Erkrankung beim Menschen in Zusammenhang gebracht wird. Bis heute ist unklar, aufgrund welcher Mechanismen die Proteinablagerungen in das Krankheitsgeschehen eingreifen. Bemerkenswert ist der von Prusiner beobachtete Zusammenhang zwischen Infektiösität und der Konzentration bestimmter Proteine, die beim Krankheitsgeschehen der Scrapie, einer neurodegenerativen Erkrankung von Schafen, eine Rolle spielen. Pathologische Proteinablagerungen treten nicht nur bei Erkrankungen des neuronalen Systems in Erscheinung, sondern werden auch in anderen Organen beobachtet, so zum Beispiel bei einer Erkrankung der Diabetes Typ II.

Eine Übersicht über Prionen-Krankheiten ist von D. Riesner in "Chemie in unserer Zeit" (1996), S. 66-74 veröffentlicht worden. Darin wird unter anderem ausgeführt, daß eine sichere und schnelle Diagnose ein vordringliches Problem der Prionenforschung darstellt, nicht nur, um biologische Sicherheit zu gewährleisten, sondern auch, um die Grundlagenforschung mit vielen offenen Fragen zur Replikation und Pathogenese von Prionen voranzutreiben. Speziell für die Alzheimersche Erkrankung ist das pathologische Bild relativ gut beschrieben. "Senile Plaques", die zum wesentlichen Teil aus aggregiertem Amyloid-β-Protein bestehen, sowie "Paired helical filaments", die aus abnormal verändertem tau-Protein aufgebaut sind, sind mit der Alzheimerschen Erkrankung eng verbunden. Heutiger Stand der Technik zur biochemischen Diagnose der Alzheimerschen Erkrankung ist die immunologische Konzentrationsmessung von löslichen Aß-Peptiden (Motter et al., Reduction of β-Amyloid peptide₁₋₄₂ in the cerebrospinal fluid of patients with Alzheimer's disease. Ann. Neurol. 38: 643; 1995) bzw. des löslichen tau-Proteins (Vadermeeren et al., Detection of tau proteins in normal and Alzheimer's disease cerebrospinal fluid with a sensitive sandwich enzyme-linked immunosorbent assay, J. Neurochemistry 61: 1828 - 1834; 1993) in der Cerebrospinalflüssigkeit (Liquor). Im U.S. Pat. No. 5,593,846 ist eine Methode beschrieben, die Konzentration von löslichem Amyloid-β-Protein zu bestimmen. Die eigentliche pathologische Komponente, die Proteinablagerungen selbst, können mit diesem Verfahren jedoch nicht gemessen werden.

Townsend fand nach Aufkonzentrierung im Liquor Strukturen, die sich mit dem seit langem bekannten, für Proteinaggregate spezifischen Farbstoff Thioflavin S anfärben lassen (Townsend et al., 1987, Neurochemical Pathology, 6, 213 - 229).

In dem entsprechenden U.S. Pat. Nr. 5,486,460 wird ein Verfahren zur Diagnose der Alzheimerschen Erkrankung beschrieben, bei dem aufkonzentrierter Liquor auf einer Glasoberfläche ausgestrichen und nach dem Eintrocknen mit Thioflavin S angefärbt wird. Dieses Verfahren ist jedoch von der praktischen Handhabung unattraktiv. Zudem ist die Färbemethode mit Thioflavin S nicht eindeutig für pathologische Proteinablagerungen, die mit der Alzheimerschen Erkrankung verknüpft sind (s.o.). Das beschriebene Verfahren fand ebenso, wie die entsprechende Publikation in der einschlägigen Fachwelt keine weitere Beachtung.

Maggio beschreibt in U.S. Pat. Nr. 5,434,050 eine Methode zur Diagnostik der Alzheimerschen Erkrankung durch Anlagerung von Aß-Peptiden an Aß-Aggregate, die als feste, gebundene Struktur, z. B. als Hirnschnittmaterial, vorliegen. Diese Diagnosemethode ist jedoch nur autoptisch praktikabel, am lebenden Patienten würde sie einen schwerwiegenden medizinischen Eingriff zur Gewinnung von Hirnbiospiematerial erfordern.

Der Wunsch, eine Messung in Körperflüssigkeiten, wie z. B. Liquor, durchzuführen, besteht daher schon länger.

Überraschenderweise wird die eingangs angesprochene Aufgabe gelöst durch ein Diagnoseverfahren mit den Merkmalen des Patentanspruchs 1.

Figur 1 zeigt ein Schema des erfindungsgemäßen Verfahrens zur Diagnostik von pathologischen Proteinablagerungen.

Figur 2 zeigt die Ergebnisse eines Experimentes zum Nachweis von Prion-Protein-Aggregaten mittels Fluoreszenz-Korrelations-Spektroskopie.

Figur 3 zeigt die Ergebnisse eines Experimentes zum Nachweis isolierter Prionen aus Gewebeproben durch fluoreszenzmarkierte solubilisierte Prion-Proteine (PrP-Cy2).

Figur 4 zeigt die Sensitivität der homologen Anlagerung von fluoreszenzmarkierten solubilisierten Prion-Proteinen (PrP-Cy2) an Prion-Protein-Aggregate aus Gewebeproben und den Einfluß der SDS-Konzentration.

Figur 5 zeigt die homologe Detektion von Peptidaggregaten, die den Großteil der amyloiden Ablagerungen bei der Alzheimerschen Erkrankung darstellen, mit fluoreszenzmarkiertem löslichen Aß(1-42)-Peptid.

Figur 6 zeigt die Ergebnisse eines Experimentes zur heterologen Detektion von Peptidaggregaten, die den Großteil der amyloiden Ablagerungen bei der Alzheimerschen Erkrankung darstellen, mit fluoreszenzmarkiertem solubilisierten Prion-Protein (PrP-Cy2).

Figur 7 zeigt die Ergebnisse eines Experimentes zum spezifischen Nachweis von Aggregaten im Liquor von Patienten, bei denen die Alzheimersche Erkrankung diagnostiziert wurde.

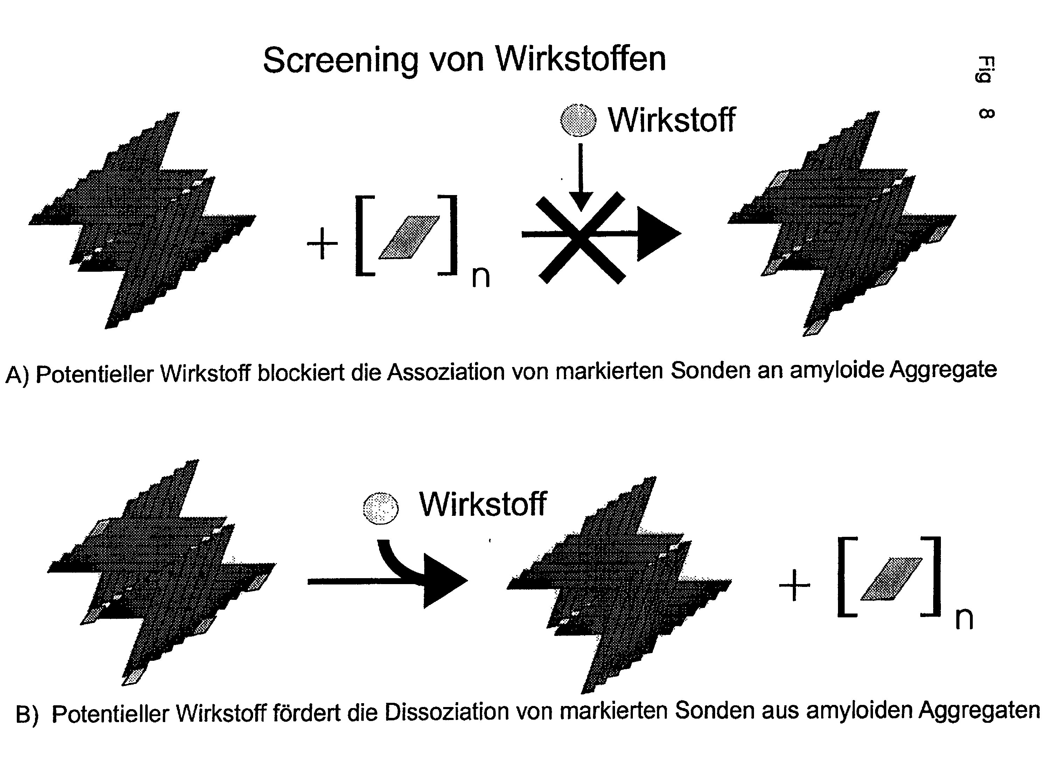

Figur 8 zeigt ein Schema zum Screening von Wirkstoffen.

Figur 9 zeigt die Kreuzkorrelationsfunktion von fluoreszenzmarkiertem Prion-Protein (90-231).

Figur 10 zeigt die Anlagerung einer Sonde von rekombinatem Prion-Protein und einem monoklonalen Antikörper an ein Prion-Protein-Aggregat.

Figur 11 zeigt die Häufigkeitsverteilung der Fluoreszenzphotonen für CJD-positive (links) und CJD-negative (rechts) Liquorproben.

Figur 12 zeigt die Häufigkeit von Fluoreszenzphotonen aus Kanälen mit mehr als 150 counts/channel in CJD-positiven und CJD-negativen Liquorproben.

Figur 13 zeigt den Einfluß von Congorot auf die Anlagerung der spezifischen Sonde Aßl-42CY2.

Das erfindungsgemäße Verfahren zur diagnostischen Erfassung von Erkrankungen umfaßt die Messung der Assoziation von Teilstrukturen der pathologischen Proteinablagerungen, pathologischen Proteinablagerungen bildende Strukturen, pathologischen Proteinablagerungen entsprechenden Strukturen und/oder pathologischen Proteinablagerungen als Sonde, an Teilstrukturen der pathologischen Proteinablagerung, pathologische Proteinablagerungen bildende Strukturen, pathologischen Proteinablagerungen entsprechende Strukturen und/oder pathologische Proteinablagerungen als Targets.

Das erfindungsgemäße Verfahren ist dadurch gekennzeichnet, daß das Target in flüssiger Phase nachgewiesen wird, wobei im Falle einer Erfassung von Alzheimer Erkrankungen die flüssige Phase aus Körperflüssigkeiten gewonnen wurde oder eine Körperflüssigkeit ist. Die Assoziation der Sonde an das Target wird gemessen, bevor eine Selbstaggregation der Sonde überwiegt.

Tritt die Assoziation der Sonde an das Target gegenüber der Selbstaggregation der Sonde in den Hintergrund, kann eine sichere Messung nicht mehr gewährleistet werden. Üblicherweise sind hierbei Meßzeiten im Minuten- und Stundenbereich möglich, was die Methode hinreichend erscheinen läßt, um in Routinelaboratorien angewendet zu werden. Die Zeiten, in denen die Messungen durchgeführt werden, hängen naturgemäß von den jeweiligen Meßbedingungen ab, sind jedoch in Vorversuchen relativ leicht zu ermitteln. Als Parameter, die die Meßzeit insbesondere beeinflußen können, sind Konzentrationen des Targets/der Sonde zu nennen. Sind beispielsweise die Sondenkonzentrationen recht hoch gegenüber der Targetkonzentration, wird die Selbstaggregation der Sonden eher einsetzen, als dies der Fall wäre, wenn die Sonden in geringer Konzentration vorhanden sind oder gar die Sondenkonzentration in ähnlicher Größenordnung wie die Targetkonzentration oder darunter liegt.

Vorzugsweise werden Meßzeiten von weniger als einer Stunde, insbesondere weniger als 30 Minuten, bevorzugt. Diese Meßzeiten sind vorteilhaft für eine Ausführung der Diagnose im Routinebetrieb.

Erfindungsgemäß weisen die Sonde und/oder das Target vorzugsweise eine detektierbare Eigenschaft auf.

Vorzugsweise sind die Teilstrukturen der pathologischen Proteinablagerungen monomere oder oligomere Einheiten pathologischer Proteinablagerungen. Die Teilstrukturen können auch homolog, insbesondere strukturhomolog, zu monomeren oder oligomeren Einheiten pathologischer Proteinablagerungen sein.

Die Sonde kann aus dem gleichen (homologe Detektion) oder einem anderen Typ (heterologe Detektion) pathologischer Proteinablagerungen stammen, welcher als Target verwendet wird. So können beispielsweise als Target eine pathologische Proteinablagerungen bildende Struktur, eine pathologischen Proteinablagerungen entsprechende Struktur und/oder pathologische Proteinablagerungen selbst, die aus einer Prionenerkrankung wie Scrapie oder BSE stammen, herangezogen werden, wohingegen die Sonde beispielsweise auch aus anderen als den genannten Strukturen stammen können. So ist es zum Beispiel möglich, die Assoziation von Teilstrukturen (Sonde), die aus amyloiden Ablagerungen aus BSE stammen, an Proteinablagerungen zu messen, die mit der Alzheimerschen Erkrankung in Verbindung stehen.

Die Teilstrukturen, die erfindungsgemäß eingesetzt werden, können vorzugsweise durch Behandlung pathologischer Proteinablagerungen mit physikalischen Methoden wie Ultraschallbehandlung, Einwirkung von Temperaturänderungen, chemischen Methoden, wie Behandlung mit Lösungen unterschiedlicher Ionenstärke, Behandlung mit Lösungen chaotroper Ionen, Behandlung mit Detergenzien und/oder Enzymen, insbesondere Proteasen, erhalten werden. So ist es beispielsweise möglich, aus an Scrapie erkranktem neuronalen Gewebe amyloide Plaques durch enzymatischen Abbau, gefolgt von Ultraschallbehandlung in Gegenwart von Detergenzien, in Teilstrukturen zu zerlegen, die nachfolgend wieder fibrilläre Strukturen aufbauen können. Die Teilstrukturen der pathologischen Proteinablagerungen können auch rekombinante Proteine, Proteinfragmente oder Peptide sein, welche Sequenzhomologien zu den entsprechenden amyloiden Plaques unterschiedlicher Herkunft und Art aufweisen können. Als Sonde kommen auch pathologischen Proteinablagerungen entsprechende Strukturen in Betracht. Solche Strukturen ahmen Bereiche der eigentlichen pathologischen Proteinablagerungen nach und treten mit den Targets assoziativ in Wechselwirkung.

Als Targets, an denen die Assoziation von Sonden gemessen wird, kommen pathologische Proteinablagerungen bildende Strukturen in Betracht. Darunter werden Strukturen verstanden, die selbst noch nicht die eigentlichen pathologischen Proteinablagerungen darstellen, sondern monomere oder oligomere Einheiten der pathologischen Proteinablagerungen oder größere Aggregate der Teilstrukturen der pathologischen Proteinablagerungen, deren Assoziation gemessen werden soll, darstellen. Als Alternative kommen auch die pathologischen Proteinablagerungen selbst in Betracht.

Die Sonde und/oder das Target weisen vorzugsweise eine detektierbare Eigenschaft auf. Die detektierbare Eigenschaft ist entweder intrinsisch in den vorgenannnten Strukturen oder Proteinablagerungen, insbesondere in der Teilstruktur vorhanden oder kann nachträglich eingeführt werden. Die mindestens eine detektierbare Eigenschaft wird insbesondere durch physikalische Methoden, vorzugsweise spektroskopisch erfaßt. Als detektierbare Eigenschaft der vorgenannten Strukturen oder Proteinablagerungen, insbesondere Teilstruktur kommt beispielsweise deren Größe oder Ausdehnung in Betracht. Auch Eigenschaften wie Struktur, meßbar mit Circular-Dichroismus, optische Eigenschaften wie Lumineszenz, Fluoreszenz oder Absorption können zur Messung der Assoziation der Sonden an die Targets herangezogen werden. So kann beispielsweise die Eigenfluoreszenz von Strukturen wie auch von nachträglich markierten Strukturen mit fluoreszierenden Eigenschaften herangezogen werden.

Zur Erhöhung der Spezifität oder Selektivität der diagnostischen Erfassung können andere, mit den Targets in Wechselwirkung tretende Substanzen eingesetzt werden. Beispielsweise ist es möglich, Substanzen, die eine Affinität zu den Targets aufweisen, beispielsweise Antikörper oder Avidin/Biotin mit den Targets in Wechselwirkung treten zu lassen.

Als Methoden zur Messung der mindestens einen detektierbaren Eigenschaft der vorgenannten Strukturen oder Anlagerungen kommen bevorzugt fluorimetrische Methoden wie konfokale Fluoreszenzspektroskopie, Fluoreszenz-Korrelations-Spektroskopie (FCS), FCS in Kombination mit Kreuzkorrelation mit jeweils entsprechenden Auswertungsverfahren in Betracht. Es wird hierbei ausdrücklich auf die DE 44 38 341 und WO-A-96/13744, die WO-A-94/16313 sowie auf die EP-A-96 116 373 und EP-A-97 109 353 verwiesen. Insbesondere die Anwendung der FCS-Kreuzkorrelation ist vorteilhaft, da durch diese Methode die Spezifität des Verfahrens verbessert werden kann. Insbesondere durch den Einsatz von verschiedenen Teilstrukturen als Sonden oder den kombinierten Einsatz mit anderen Sonden für pathologische Proteinablagerungen, wie z.B. spezifische Antikörper, können falsch positive Detektionen von Aggregaten in biologischen Medien unterdrückt werden.

Es kann bevorzugt sein, zur Erhöhung der Sensitivität der Detektion und zur Steigerung der Analysengeschwindigkeit auf ein "Abrastern" der Meßlösung über ein durch die Meßlösung bewegtes konfokales Volumenelement zurückzugreifen.

Bei der Kreuzkorrelation werden jeweils zwei unterschiedlich fluoreszierende Spezies beobachtet.

Die detektierbaren Eigenschaften werden beispielsweise durch Fluoreszenzmarkierungen, die niedermolekulare, aber auch hochmolekulare Gruppen sein können, hergestellt. So können beispielsweise markierte Antikörper, die an die Targets gebunden werden, letztere markieren. Das Binden von Sonden kann dann entsprechend vermessen werden. Es können verschiedenfarbige Markierungen unterschiedlicher Sonden vorgenommen werden und an diesen eine Kreuzkorrelation durchgeführt werden. Es ist ebenfalls möglich, die Sonden mit entsprechenden Labeln zu versehen, die entweder niedermolekulare fluoreszierende Liganden und/oder entsprechende Markierungskonjugate sind.

Erfindungsgemäß stammen die pathologischen Proteinablagerungen vorzugsweise aus amyloiden Plaques, die mit neurodegenerativen Erkrankungen wie der Alzheimerschen Erkrankung, boviner spongioformer Enzephalopathie (BSE), Creutzfeldt-Jakob-Erkrankung, Scrapie (Traberkrankheit), Chorea-Huntington, Morbus Parkinson einhergehen, oder aus amyloiden Plaques anderer Organe als dem neuronalen System, wie zum Beispiel mit Diabetes verbundenen Proteinablagerungen. Eine geeignete Quelle für die pathologischen Proteinablagerungen stellen Organextrakte, bevorzugt Hirnextrakte erkrankter Tiere, dar. Diese können beispielsweise mit Prionen infizierte Syrische Goldhamster sein. Die folgende Übersicht gibt Erkrankungen wider, welche mit amyloiden Erscheinungen verbunden sind:

| | | |
|---|---|---|
| Amyloide Erkrankung | Vorkommen | Amyloid-bildendes Protein |
| Morbus Alzheimer | neuronal | Amyloid-β-Protein |
| Transmissible Spongiforme Encephalopathien | neuronal | Prion-Protein |
| Chorea Huntington* | neuronal | Huntingtin* |
| Morbus Parkinson* | neuronal | Synuclein* |
| Vererbbare Cebrale Amyloide Angiophatie | neuronal | Cystatin C |
| Primäre Aystemische Amyloidose (AL-Amyloidose) | systemisch | Immunoglobulin |
| Reaktive Aystemische Amyloidose (AA-Amyloidose) | systemisch | Lipoproteine |
| Familiäre Amyloide Polyneuropathie | systemisch | Transthyretin |
| Typ II Diabetes | Pankreas | Islet-Amyloid-Polypeptid |
| Injektions-Lokalisierte Amyloidose | | Insulin |
| Medullaria-Carcinom der Schilddrüse | Schilddrüse | Calcitonin |
| Beta-2-Microglobulin Amyloidose | Skelettmuskel | Beta-2-Microglobulin |
| Vererbte Nichtneuropatische Amyloidose | systemisch | Lysozym |
| Finnische Vererbte Systemische Amyloidose | systemisch | Gelsolin |

| | | |
|---|---|---|
| (* verändert nach Sipe, 1992 Annual Reviews in Biochemistry 61; 947-975) | | |

Die Teilstrukturen der pathologischen Proteinablagerungen können auch rekombinante Proteine, Proteinfragmente oder Peptide sein, welche Sequenzhomologien zu amyloiden Plaques aufweisen. So können beispielsweise konservative Aminosäuresubstitutionen in hochkonservierten Regionen wie folgt berücksichtigt werden: Jede Isoleucin-, Valin- und Leucin-Aminosäure kann gegen eine andere dieser Aminosäuren ausgetauscht sein, Aspartat kann gegen Glutamat und umgekehrt ausgetauscht sein, Glutamin gegen Asparagin und umgekehrt, Serin gegen Threonin und umgekehrt. Konservative Aminosäuresubstitutionen in weniger hochkonservierten Regionen können wie folgt sein: Jede der Aminosäuren Isoleucin, Valin und Leucin kann gegen jede andere dieser Aminosäuren, Aspartat gegen Glutamat und umgekehrt, Glutamin gegen Asparagin und umgekehrt, Serin gegen Threonin und umgekehrt, Glycin gegen Alanin und umgekehrt, Alanin gegen Valin und umgekehrt, Methionin gegen jede der Aminosäuren Leucin, Isoleucin oder Valin, Lysin gegen Arginin und umgekehrt, eine der Aminosäuren Aspartat oder Glutamat gegen eine der Aminosäuren Arginin oder Lysin, Histidin gegen eine der Aminosäuren Arginin oder Lysin, Glutamin gegen Glutamat und umgekehrt und Asparagin gegen Aspartat und umgekehrt, ausgetauscht sein.

Als Quelle für Material, das einer diagnostischen Untersuchung mit dem erfindungsgemäßen Verfahren unterworfen wird, kommen insbesondere Körperflüssigkeiten wie Liquor cerebrospinalis, Blut, Lymphe, Urin oder Sekrete wie Sputum in Frage. Aus den genannten Körperflüssigkeiten oder Sekreten werden Proben entnommen und zur Messung der Assoziation von potentiell in diesen Proben enthaltenen pathologische Proteinablagerungen bildenden Strukturen, pathologischen Proteinablagerungen entsprechenden Strukturen und/oder pathologischen Proteinablagerungen, mit Sonden in Kontakt gebracht und inkubiert. Das Vorhandensein der pathologischen Proteinablagerungen, die indikativ für eine damit verbundene Erkrankung sind, ergibt dann ein positives diagnostisches Signal, vermittelt durch die Assoziation der zugesetzten Sonden. Körperflüssigkeiten sind gegenüber Geweben vorteilhaft, da hier eine direkte Inkubation erfolgen kann, wohingegen Gewebe regelmäßig erst aufgeschlossen werden müssen. Dies kann beispielsweise durch mechanische oder chemische Behandlung oder Kombinationen davon erfolgen.

Als Parameter, die aus den detektierbaren Eigenschaften der genannten Strukturen und Ablagerungen, insbesondere Teilstrukturen ermittelt werden, kommen insbesondere Translationsdiffusionsgeschwindigkeiten, Rotationsdiffusionsgeschwindigkeiten, Lebensdauern angeregter Zustände, Polarisation von Strahlung, Energietransfer, Quantenausbeute, Partikelzahl oder Konzentration, Intensitätsdifferenzen in Betracht. Wird die Translationsdiffusionsgeschwindigkeit ermittelt, so kann es bei langsam diffundierenden Assoziaten wünschenswert sein, diese mittels eines Scanningprozesses mehrfach zu erfassen.

Die Belastung für den Patienten durch Lumbalpunktion zur Gewinnung von Liquor ist gering. Eine deutliche Sensitivitätssteigerung kann bei Einbeziehung des Prozesses der Keim-induzierten Polymerisation erzielt werden, wie er von Jarret und Lansbury (Cell 73, 1055-1058, 1993) für amyloide Erkrankungen theoretisch beschrieben worden ist. In diesem Modell wird die Aggregation von amyloiden Ablagerungen in zwei kinetische Reaktionen unterteilt. Bei der Selbstaggregationskinetik von amyloiden Proteinen zu oligomeren Untereinheiten liegt das Gleichgewicht deutlich auf der Seite der Edukte. Aggregate entstehen erst nach einer längeren Reaktionszeit. Im Anschluß daran kommt es zu einer Keim-abhängigen Polymerisation, bei der sich amyloide Proteine an die schon gebildeten Aggregate anlagern. Hier liegt das Gleichgewicht deutlich auf Seiten der Produkte. Die Anlagerung findet hier ohne Zeitverzögerung statt. Dieses Zeitfenster zwischen den beiden kinetischen Reaktionen sollte sich für die Detektion von natürlichen Proteinablagerungen nutzen lassen. Nach Zugabe einer Sonde, die einen Polymerisationsprozeß unterstützen kann, sollte im Falle des Vorhandenseins von pathologischen Proteinablagerungen, die als Keime dienen, ein deutlich schnellerer Einbau der Sonde in diese Proteinablagerung erfolgen, als die Bildung der durch langsamere Selbstaggregation der Sonde entstehenden Aggregate, die jedoch unter Umständen nicht von ersteren Aggregaten unterscheidbar sind.

Insbesondere die Kombination der Keim-induzierten Anlagerung einer Vielzahl von markierten Sonden an als Keime dienende pathologische Proteinablagerungen mit dem extrem sensitiven Nachweisverfahren mittels konfokaler Optik ergibt eine dementsprechend große Verstärkung des Meßsignals. Im durch Lumbalpunktion (Neumeister et al., Klinikleitfaden Labordiagnostik, Fischer-Verlag 1998) gewonnenen Liquor konnten so einzelne Moleküle der pathologischen Proteinablagerungen eindeutig detektiert werden.

Ein Screening-Verfahren zum Aufspüren von Wirkstoffen zur Behandlung von mit pathologischen Proteinablagerungen verbundenen Erkrankungen, das dem erfindungsgemäßen Verfahren analog ist, geht davon aus, daß in Gegenwart von vermuteten Wirkstoffen die Assoziation von Teilstrukturen der pathologischen Proteinablagerungen, pathologische Proteinablagerungen bildende Strukturen, pathologische Proteinablagerungen entsprechenden Strukturen und/oder pathologischen Proteinablagerungen, an Teilstrukturen der pathologischen Proteinablagerung, pathologische Proteinablagerungen bildende Strukturen, pathologischen Proteinablagerungen entsprechende Strukturen und/oder pathologische Proteinablagerungen als Targets gemessen wird. Als Alternative wird zunächst eine Assoziation von Teilstrukturen der pathologischen Proteinablagerungen, pathologische Proteinablagerungen bildende Strukturen, pathologische Proteinablagerungen entsprechenden Strukturen und/oder pathologischen Proteinablagerungen, an Teilstrukturen der pathologischen Proteinablagerung, pathologische Proteinablagerungen bildende Strukturen, pathologischen Proteinablagerungen entsprechende Strukturen und/oder pathologische Proteinablagerungen als Targets durchgeführt und dann die Umkehrung der Assoziation unter Einwirkung von vermuteten Wirkstoffen beobachtet. Als Wirkstoff werden dann Verbindungen identifiziert, die in der Lage sind, eine Umkehrung der Assoziation in signifikanter Weise zu bewirken. Eine dritte Alternative besteht darin, daß die Dissoziation von Proteinablagerungen selbst unter Einwirkung von vermuteten Wirkstoffen gemessen wird.

Das erfindungsgemäße Verfahren wird durch die nachfolgenden Beispiele näher erläutert.

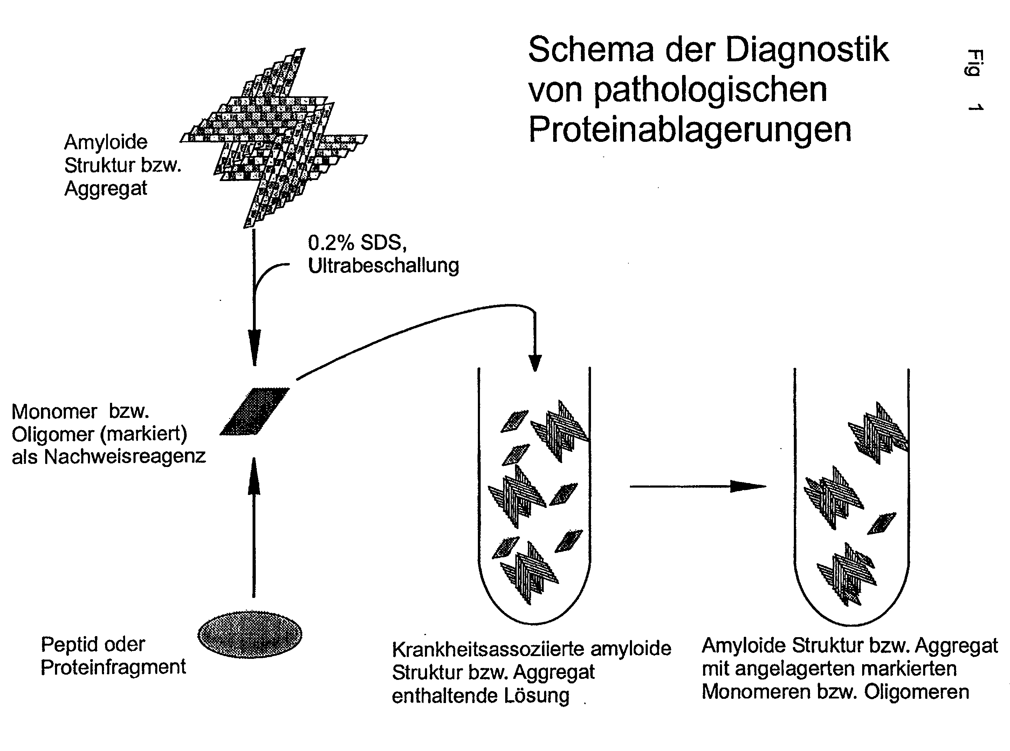

Das in Figur 1 aufgeführte Schema zeigt das experimentelle Vorgehen für die nachfolgenden Beispiele 1 bis 6, 8 bis 10.

Die Versuche 1 bis 3 und 5 wurden mit solubilisiertem Prion-Proteinen durchgeführt, während die Versuche 7 bis 10 rekombinantes Prion-Protein (90-231) betreffen.

Die Präparation von löslichen (solubilisierten) Prion-Proteinen (PrP) erfolgte durch Ultrabeschallung in 10 mM Natrium-Phosphat pH 7,2, 0,20% SDS von Prion-Aggregaten, isoliert aus infektiösem Hirngewebe des Syrischen Goldhamsters (Riesner, D.; Kellings, K.; Post, K.; Wille, H.; Serban, H.; Groth, D.; Baldwin, M. A. and Prusiner, S. B., 1996, Journal of Virology, Vol. 70, Nr. 3, Seiten 1714 - 1722). In Abhängigkeit von der Energie der Ultrabeschallung entsteht eine monomere bis oligomere PrP-Fraktion, die für die folgenden beschriebenen Beispiele (1 bis 3, 5) fluoreszenzmarkiert wurde (Pitschke, M.; Post, K. and Riesner, D, 1997, Progress in Colloid & Polymer Science, Vol. 107, Seiten 72 - 76). Zur Fluoreszenzmarkierung wurde der Farbstoff Cy2 (Amersham) verwendet.

Lösliches Aß(1-42) konnte in 10 mM Na-Phosphatpuffer, pH 7,0 mit 0,2% SDS stabilisiert werden (Beispiel 4, 6). Zur Fluoreszenzmarkierung von Aß wurde ebenfalls der Farbstoff Cy2 (Amersham) verwendet.

Für Assoziationsmessungen wurden die löslichen, monomeren bis oligomeren fluoreszenzmarkierten Proteine bzw. Peptide unter Ausdünnung des SDS auf 0,02% SDS mit amyloiden Aggregaten (infektiöse Prion-Partikel: Beispiele 1 bis 3; Aß: Beispiele 4 bis 5) inkubiert und das Fluoreszenzsignal mittels Fluoreszenz-Korrelations-Spektroskopie detektiert.

### Beispiel 1

### Nachweis von Prion-Protein-Aggregaten mittels Fluoreszenz-Korrelations-Spektroskopie (FCS)

Detektion von infektiösen Prion-Partikeln (PrP) mittels FCS durch Anlagerung von fluoreszenzgelabelten solubilisierten PrP.

### Material:

- solubilisiertes PrP-Cy2, ca. 25 ng/µl in 10 mM Na-Phosphatpuffer, pH 7,0, 0,2% SDS => (Lsg. 1)
- Prion-Partikel (ca. 200 ng/µl) in 10 mM Na-Phosphatpuffer, pH 7,0 => (Lsg. 2)

### Durchführung:

Es wurden zwei Ansätze hergestellt:

| | | | |
|---|---|---|---|
| A) | 1 µl Lsg. 1 (1 : 10) 19 µl Na-Phosphatpuffer, pH 7,0 | B) | 1 µl Lsg. 1 (1 : 10) 17 µl Na-Phosphatpuffer, pH 7,0 2 µl Lsg. 2 (1 : 100) |

Beide Ansätze wurden gemischt, für 1 Minute inkubiert, in eine Probenkammer appliziert und in FCS gemessen.

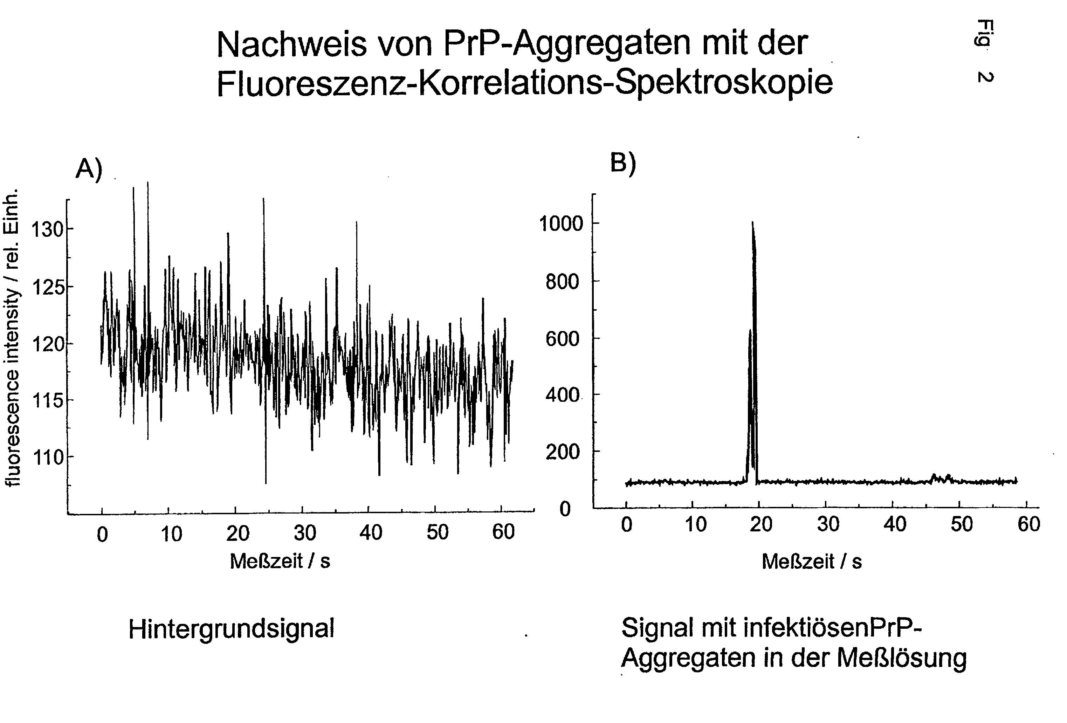

Figur 2 zeigt das Ergebnis der Messung der Fluoreszenzintensität (in relativen Einheiten/RE) über einen Zeitraum von 60 Sekunden. In Figur 2a erkennt man das Fluktuationssignal des solubilisierten PrP-Cy2, das eine Signalintensität zwischen 110 und 130 RE aufweist. Eine Auswertung des Signals mittels Autokorrelation ergibt eine Diffusionszeit von ca. 250 µs, die typischerweise dem monomeren Protein entspricht. In Figur 2b ist zusätzlich ein Signalpeak mit einer Fluoreszenzintensität von ca. 950 RE zu erkennen, der durch die langsame Diffusion eines größeren Aggregates, an welches das vormals solubilisierte fluoreszenzmarkierte PrP-Cy2 angelagert ist, verursacht wird. Da es sich nur um ein einmaliges Ereignis handelt, ist eine Bestimmung der Diffusionszeit bzw. des Molekulargewichts mittels Autokorrelation nicht möglich. Eine eindeutige Signaldetektion und Zuordnung zu einzelnen Aggregaten gelingt jedoch über das Auftreten isolierter Peaks mit drastischer Erhöhung der Fluoreszenzintensität.

### Beispiel 2

### Homologe Detektion von isolierten Prionen aus Gewebeproben mit fluoreszenzmarkierten solubilisierten Prion-Proteinen (PrP-Cy2)

Detektion von infektiösen Aggregaten der Prion-Proteine (PrP-Rods) in Hirnextrakten nach Markierung mit fluoreszenzgelabelten solubilisierten Prion-Proteinen (PrP-Cy2) mittels FCS.

### Material:

- solubilisiertes PrP-Cy2, ca. 25 ng/µl in 10 mM Na-Phosphatpuffer, pH 7,0, 0,2% SDS => (Lsg. 1)
- Hirnextrakt aus nicht infizierten (Lsg. 2) und Prioninfizierten Syrischen Goldhamstern (Lsg. 3)

### Durchführung:

Es wurden zwei Ansätze hergestellt:

| | | | |
|---|---|---|---|
| A) | 1 µl Lsg. 1 (1 : 10) 17 µl Na-Phosphatpuffer, pH 7,0 2 µl Lsg. 2 (1 : 10) | B) | 1 µl Lsg. 1 (1 : 10) 17 µl Na-Phosphatpuffer, pH 7,0 2 µl Lsg. 3 (1 : 10) |

Beide Ansätze wurden gemischt, für 1 Minute inkubiert, in eine Probenkammer appliziert und im FCS gemessen.

Als Kontrolle wurden solubilisierte PrP-Cy2 ohne Zugabe von Hirnextrakt in gleicher Konzentration vermessen.

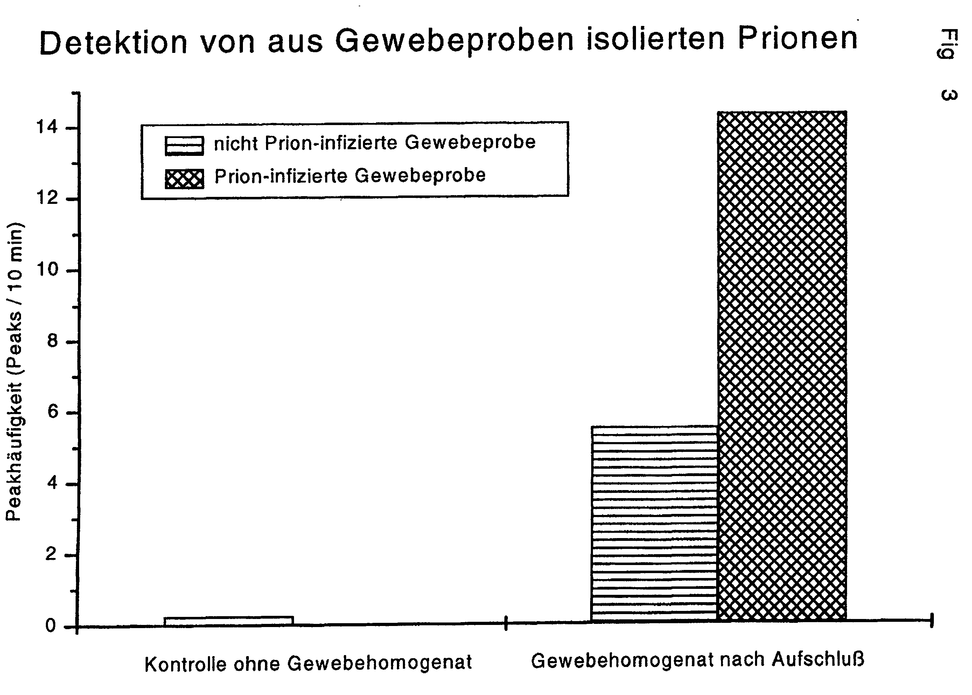

In Figur 3 ist als graphische Darstellung die Peakfrequenz für die einzelnen Ansätze aufgetragen. Dabei wurde die Anzahl der Peaks, verursacht durch Diffusion hochmolekularer Aggregate durch das konfokale Volumenelement (siehe Beispiel 1), detektiert. Zu erkennen ist, daß die prioninfizierte Gewebeprobe eine deutlich höhere Peakfrequenz gegenüber der Probe mit nicht infiziertem Hirnextrakt aufweist.

Eine Detektion von infektiösem Material durch Anlagerung von solubilisiertem PrP-Cy2 an vorhandene Prionen ist mit dieser Versuchsanordnung möglich, wenn auch die Peakfrequenz bei Untersuchungen von nicht-infizierter Gewebeprobe einen Untergrund zeigt.

### Beispiel 3

### Sensitivität der homologen Anlagerung von fluoreszenzmarkierten solubilisierten Prion-Proteinen (PrP-Cy2) und aus Gewebeproben isolierten Prion-Protein-Aggregaten

Die Sensitivität der homologen Anlagerung von PrP-Cy2 an aus infektiösem Hirngewebe isolierten Prionen wird mit FCS analysiert.

### Material:

- solubilisiertes PrP-Cy2, ca. 25 ng/µl in 10 mM Na-Phosphatpuffer, pH 7,0, 0,2% SDS => (Lsg. 1)
- PrP-Rods (ca. 200 ng/µl) in 10 mM Na-Phosphatpuffer pH 7,0 => (Lsg. 2)

### Durchführung:

Es wurden zwei Versuchsreihen hergestellt:

| | | | |
|---|---|---|---|
| A) | 1 µl Lsg. 1 (1 : 10) 17 µl Na-Phosphatpuffer, pH 7,0, 2 µl Lsg. 2 (1 : x; PrP-Menge, s. Figur 4) | B) | 1 µl Lsg. 1 (1 : 10) 17 µl Na-Phosphatpuffer, pH 7,0, 0,2% SDS 2 µl Lsg. 2 (1 : x, (PrP-Menge s. Figur 4) |

Die Menge der PrP-Rods im Versuchsansatz wurde dabei schrittweise bis zur Nachweisgrenze abgesenkt. Die Messung in 0,2% SDS dient dabei als Kontrolle.

Alle Ansätze wurden gemischt, für 1 Minute inkubiert, in eine Probenkammer appliziert und mittels FCS gemessen.

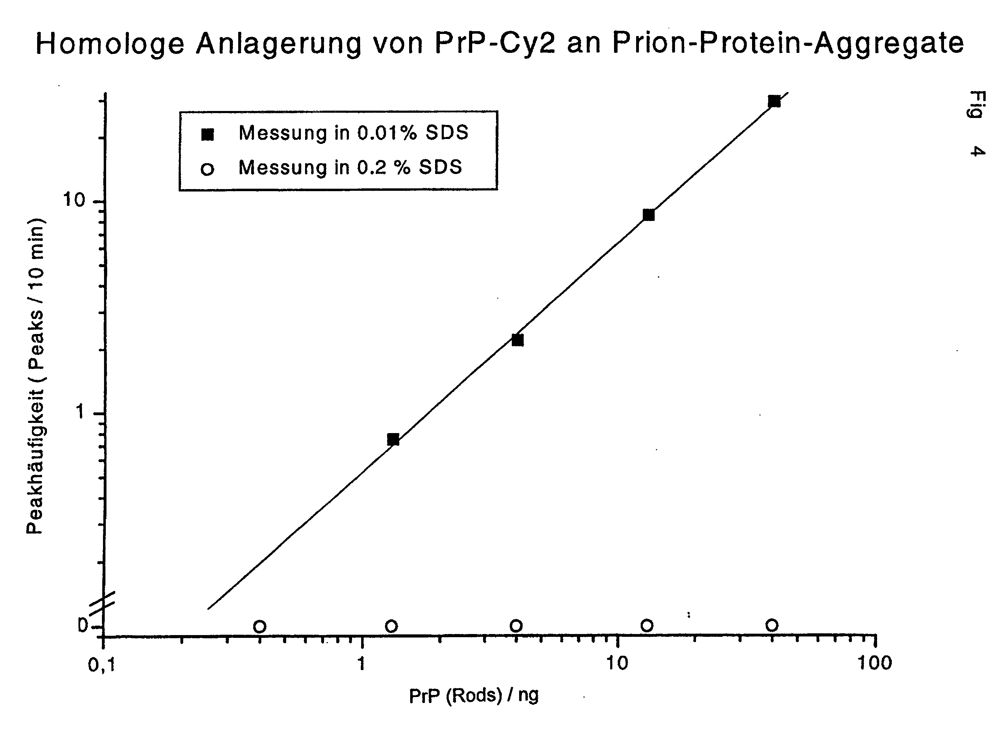

Die Peakfrequenz, ausgedrückt in Peaks pro 10 min Meßzeit wurde in Figur 4 in Abhängigkeit von der PrP-Menge im Versuchsansatz aufgetragen. Über den gemessenen Konzentrationsbereich von 40 ng bis 400 pg ist eine direkte lineare Abhängigkeit zur Peakfrequenz zu erkennen. Die Auswertung der Anzahl der Peaks läßt dadurch eine direkte Quantifizierung der Prionkonzentration in der zu vermessenden Probe zu. In 0,2% SDS ist kein Einbau in schon existierende PrP-Strukturen möglich.

### Beispiel 4

### Homologe Detektion von Peptidaggregaten, die den Großteil der amyloiden Ablagerungen bei der Alzheimerschen Erkrankung darstellen, mit fluoreszenzmarkiertem löslichen Aß(1-42)-Peptid

Detektion von Aggregaten der Alzheimerschen Erkrankung mit fluoreszenzgelabeltem löslichen Aß(1-42) mittels FCS mit der Fragestellung, ob das in den Beispielen 1 bis 3 eingesetzte Detektionsverfahren auch auf andere Erkrankungen mit pathologischen Proteinablagerungen übertragbar ist und damit zum Nachweis jeder amyloiden Struktur geeignet ist.

### Material:

- lösliches Aß Peptid 1-42, fluoreszenzmarkiert mit Cy2, 100 ng/µl in 10 mM Na-Phosphatpuffer, pH 7,0, 0,2% SDS => (Lsg. 1)
- Aß-Amyloidfibrillen (90 ng/µl) in 10 mM Na-Phosphatpuffer pH 7,0 => (Lsg. 2)

### Durchführung:

Es wurden zwei Ansätze hergestellt:

| | | |
|---|---|---|
| A) | 1 µl Lsg. 1 (1 : 10) B) 10 µl Lsg. 2 9 µl Na-Phosphatpuffer, pH 7,0 | 1 µl Lsg. 1 (1 : 10) 10 µl Lsg. 2 9 µl Na-Phosphatpuffer, 0,2% SDS, pH 7,0 |

Beide Ansätze wurden gemischt, für 1 min inkubiert, in eine Probenkammer appliziert und im FCS gemessen.

Als Kontrolle wurde lösliches Aß(1-42)-Cy2 in gleicher Konzentration vermessen.

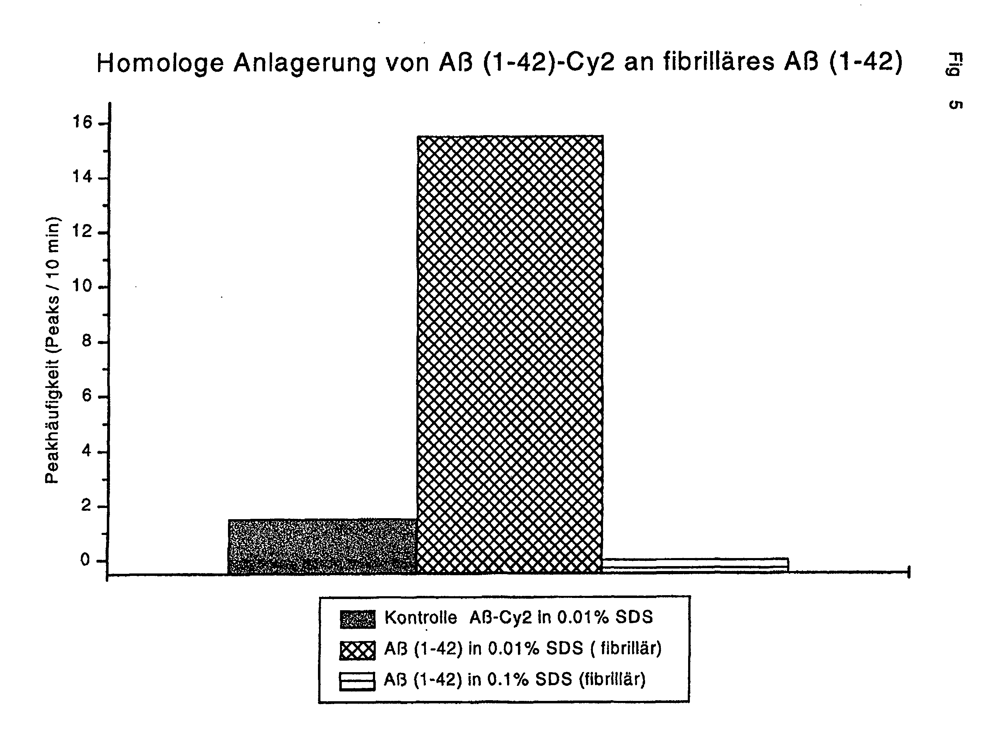

In Figur 5 sind die Ergebnisse des Experiments graphisch zusammengefaßt. Die Auswertung der Peakfrequenz (vgl. 1 - 3) zeigt, daß bei Inkubation in 0,01% SDS eine Markierung von Aß-Fibrillen (SDS-Konzentration identisch wie bei PrP-Aggregaten), die die Hauptkomponenten der amyloiden Ablagerungen bei der Alzheimerschen Erkrankung darstellen, nach diesem Verfahren möglich ist. Ein Einbau von Aß-Cy2 (1-42) in Gegenwart einer erhöhten SDS-Konzentration (0,1%) ist nicht möglich. Auch eine Markierung von Aß, das in löslicher (nicht fibrillärer) Struktur vorliegt, ist nicht möglich. Das Experiment zeigt, daß eine Detektion von amyloiden Ablagerungen einhergehend mit der Alzheimerschen Erkrankung möglich ist.

### Beispiel 5

### Heterologe Detektion von Peptidaggregaten, die den Großteil der amyloiden Ablagerungen bei der Alzheimerschen Erkrankung darstellen, mit fluoreszenzmarkierten solubilisierten Prion-Proteinen (PrP-Cy2)

Detektion von Aggregaten der Alzheimerschen Erkrankung mit fluoreszenzgelabeltem löslichem PrP-Cy2 mittels FCS mit der Fragestellung, ob auch eine heterologe Markierung mit einer fluoreszenzmarkierten löslichen Sonde möglich ist, die nicht identisch mit der Probe ist, an die eine Anlagerung stattfinden soll.

### Material:

- solubilisiertes PrP-Cy2, ca. 25 ng/µl in 10 mM Na-Phosphatpuffer pH 7,0, 0,2% SDS => (Lsg. 1)
- Aß(1-42) Amyloidfibrillen (90 ng/µl) in 10 mM Na-Phosphatpuffer pH 7,0 => (Lsg.2)
- Aß(1-42) nicht fibrillär (90 ng/µl) in 10 mM Na-Phosphatpuffer pH 7,0, 0,2% SDS => (Lsg. 3)
- Aß(1-40) Amyloidfibrillen (90 ng/µl) in 10 mM Na-Phosphat pH 7,0 => (Lsg. 4)
- Aß(1-40) nicht fibrillär (90 ng/µl) in 10 mM Na-Phosphatpuffer pH 7,0, 0,2%, SDS => (Lsg. 5)

### Durchführung:

Es wurden folgende Ansätze hergestellt:

| Aß-Peptid(1-42) : | | |
|---|---|---|
| A) | 1 µl Lsg. 1 (1 : 10) B) 2,5 µl Lsg. 2 16,5 µl Na-Phosphatpuffer, pH 7,0 | 1 µl Lsg. 1 (1 : 10) 2,5 µl Lsg. 3 16,5 µl Na-Phosphatpuffer, pH 7,0 |
| C) | 1 µl Lsg. 1 (1 : 10) 2,5 µl Lsg. 2 16,5 µl Na-Phosphatpuffer, pH 7,0, 0,2% SDS | |

| Aß-Peptid(1-40) : | | |
|---|---|---|
| A) | 1 µl Lsg. 1 (1 : 10) B) 2,5 µl Lsg. 4 16,5 µl Na-Phosphatpuffer, pH 7,0 | 1 µl Lsg. 1 (1 : 10) 2,5 µl Lsg. 5 16,5 µl Na-Phosphatpuffer, pH 7,0 |
| C) | 1 µl Lsg. 1 (1 : 10) 2,5 µl Lsg. 4 16,5 µl Na-Phosphatpuffer, pH 7,0, 0,2% SDS | |

Alle Ansätze wurden gemischt, für 1 min inkubiert, in eine Probenkammer appliziert und im FCS gemessen.

Als Kontrolle wurde lösliches PrP-Cy2 in gleicher Konzentration bei 0,01% SDS vermessen.

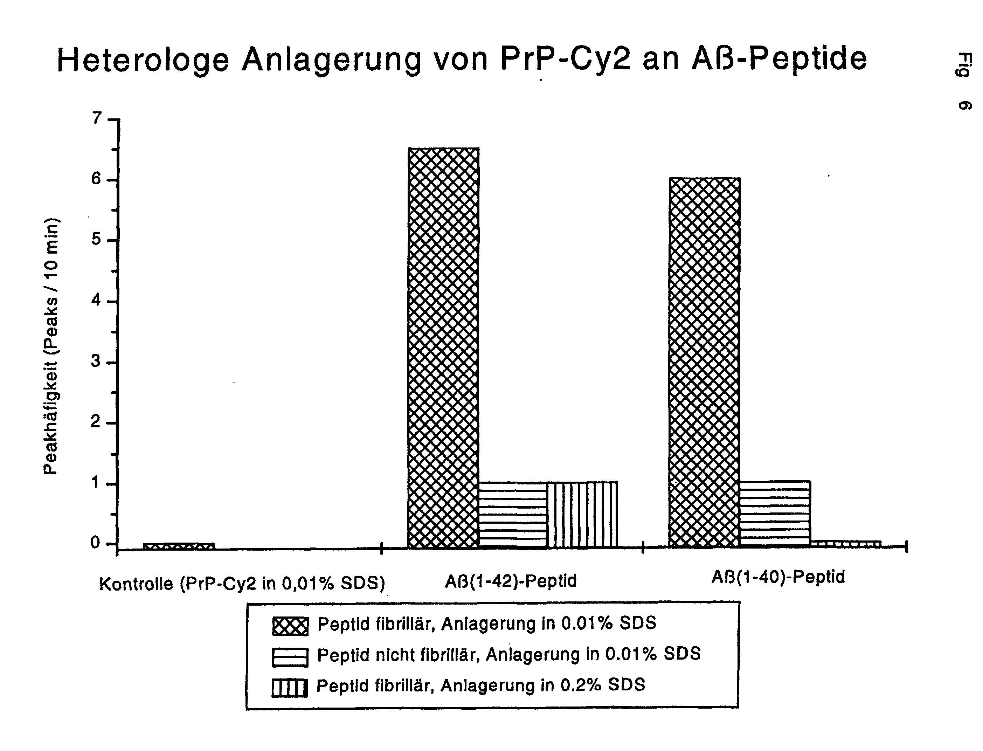

Für jeden der oben aufgeführten Ansätze wurde die Peakfrequenz (vgl. Beispiel 2) gemessen (Figur 6). Dabei zeigt sich, daß sowohl Aß(1-42) als auch Aß(1-40) deutlich mit PrP-Cy2 markiert werden können, wenn sie in fibrillärer Struktur vorliegen, und die Markierungsreaktion bei 0,01% SDS stattfindet. Bei Anlagerung an nicht-fibrilläre Strukturen sowie erhöhter SDS-Konzentration ist nur ein geringes Hintergrundsignal zu erkennen.

Dieses Ergebnis zeigt, daß es für eine Markierung amyloider Proteinaggregate nicht zwingend notwendig ist, daß das zur Markierung eingesetzte lösliche fluoreszenzmarkierte Protein identisch mit dem aggregatbildenden Protein sein muß.

### Beispiel 6

### Nachweis von Aggregaten im Liquor von Patienten, die an der Alzheimerschen Erkrankung leiden

Spezifische Detektion von Aggregaten, die mit der Alzheimerschen Erkrankung verbunden sind. Dazu wird Liquor (Rückenmarksflüssigkeit) als Probe verwendet, in dem spezifisch zwischen Patienten, die an der Alzheimerschen Erkrankung leiden und einer gesunden Kontrollgruppe unterschieden werden kann.

### Material:

- lösliches Aß-Peptid 1-42, fluoreszenzmarkiert mit Cy2, 100 ng/µl in 10 mM Na-Phosphatpuffer pH 7,0, 0,2% SDS => (Lsg. 1)
- Liquor aus erkrankten Patienten und Kontrolle aus nicht erkrankten Patienten

### Durchführung:

Es wurden folgende Ansätze hergestellt:
2 µl Lsg. 1 (1 : 10)
18 µl Patientenliquor

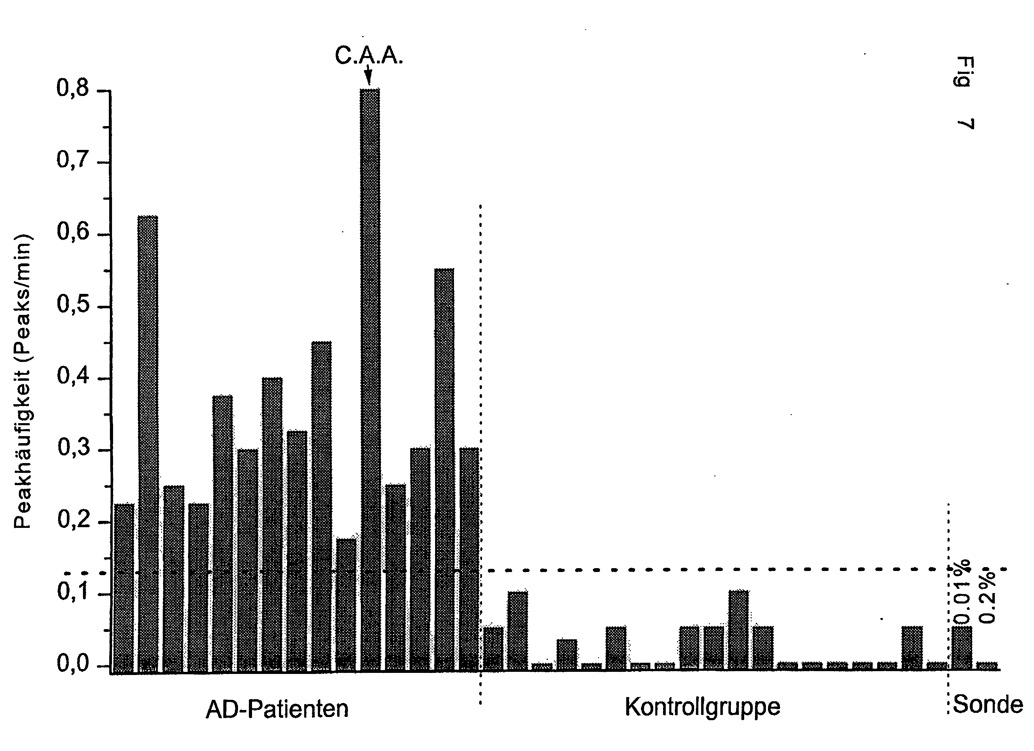

Die Liquorproben wurden direkt und ohne weitere Behandlung bzw. Aufkonzentrierung mit fluoreszenzmarkiertem Aß1-42-Cy2 (0,1 ng/µl) bei 0,02% SDS inkubiert und sofort für 20 min mittels FCS vermessen (Fig. 7). Die detektierten Aggregate in Liquorproben von nach psychiatrischen Diagnostikgesichtspunkten an der Alzheimerschen Demenz (AD) erkrankten Patienten sowie nicht erkrankten Kontrollpatienten wurden verglichen. Dabei zeigt sich, daß alle an Alzheimerscher Demenz erkrankten Patienten eine signifikant höhere Signalintensität im Liquor aufweisen, als die entsprechende Kontrollgruppe, die eine ähnliche Alterszusammensetzung aufwies. Der mit CAA bezeichnete Patient weist von allen diagnostizierten Patienten die höchste Signalintensität auf. Dieser Patient ist an congophiler Angiopathie erkrankt, einer Sonderform der Alzheimerschen Erkrankung, bei der eine Ablagerung von Amyloid-β-Peptiden in den Kapillaren der Hirngefäße stattfindet, deren Wanderung dadurch sukzessive zerstört wird. Bei dieser Erkrankung scheint der massivste Übertritt von Aß-Aggregaten in den Liquor stattzufinden, was zu dem starken Anstieg der Signalintensität führt. Die Meßergebnisse der Kontrollgruppe zeigen, daß auch hier vereinzelt Aggregate detektiert werden. Dieses Ergebnis zeigt auch die Sonde ("probe") alleine (Aß 1-42-Cy2) und beruht auf Selbstaggregationseffekten während der 20-minütigen Meßzeit oder auf schon vorhandenen Aß-Aggregaten. Die Meßergebnisse der Sonde wurden in 0,01% bzw. 0,2% SDS erzielt.

### Beispiel 7

### Koaggregation zweier fluroeszenzmarkierter rPrP-Sonden an rPrP (90-231)

Rekombinantes Prion-Protein (90-231) (nachfolgend rPrP genannt), das homolog zum proteaseresistenten Teil von Hamster PrP ist, wurde mit aminoreaktiven Fluoreszenzfarbstoffen der Anregungswellenlängen 488 nm (Oregon Green, Molecular Probes) und 633 nm (Cy5, Amersham) markiert. Proteinkonzentrationen und Markierungsverhältnis wurden durch Absorptionsmessung bei 280 nm, 496 nm und 650 nm bestimmt. Die Markierungseffizienz lag bei 10% (rPrP-Cy5) bzw. 4% (rPrp-OrG) bei äquimolarem Zusatz des aminoreaktiven Fluoreszenzfarbstoffs.

Aggregationsexperimente: Äquimolare Mengen beider markierter rPrP Sonden wurden mit einem 25fachen Überschuß von unmarkiertem rPrP gemischt, wobei der Puffer 0,2% SDS enthielt. Durch Verdünnung auf eine SDS-Konzentration von 0,01% und eine rPrP-Gesamtkonzentation von 50 nM wurde der Aggregationsprozeß gestartet.

Der Aggregationsprozeß wurde durch wiederholte Aufnahme von Auto- und Kreuzkorrelationskurven in einem Zweifarben-Kreuzkorrelations FCS Gerät (ConfoCor Prototyp, Zeiss) verfolgt. Die Korrelationskurven wurden durch ein dreidimensionales Diffusionsmodell modeliert, wobei für die Kreuzkorrelationskurven ein Diffusionsparameter τrg verwendet wurde. Es wurde mit einem Modell für kugelförmige Moleküle angefittet.

Die Erniedrigung der SDS-Konzentration bewirkt einen schnellen Anstieg der Kreuzkorrelationsamplitude, die zur Konzentration der Teilchen proportional ist, in die sowohl rot als auch grün markiertes PrP inkorportiert wurde. In Anwesenheit von 0,2% SDS konnte kein Kreuzkorrelationsprodukt nachgewiesen werden. Die Oligomerkonzentration steigt in wenigen Minuten bis zu einem Plateau und nimmt danach durch die Bildung höherer Aggregate langsam ab.

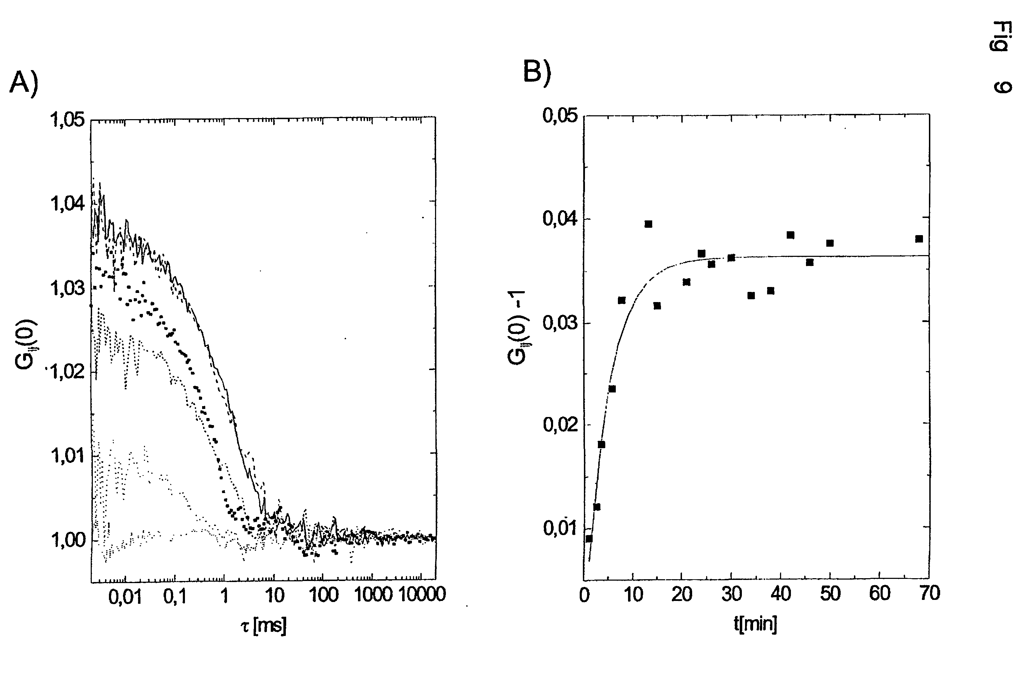

Figur 9 (links) zeigt die Kreuzkorrelationsfunktion von fluoreszenzmarkiertem rPrP(90-231), c(rPrP) = 50 nM, c(rPrP-Cy5), c(rPrP-OrG) = 2 nM in PBS, 25°C + 0,01% SDS. Es bedeuten dabei a) kleinere Punkte: 1 min Inkubationszeit; b) kurze Striche: 6 min; c) stärkere Punkte: 20 min; d) gestrichelte Linie: 30 min; e) durchgezogene Linie: 130 min; f) kleine Punkte: Referenzprobe 0,2% SDS.

Figur 9 (rechts) zeigt den Plot der Kreuzkorrelationsamplitude im Laufe der Aggregation (squares). Die Bildung von Partikeln, die sowohl rote als auch grüne Fluorophore inkorporiert haben, läßt sich durch eine Reaktion zweiter Ordnung beschreiben: Aᵢ + Bⱼ -> ABᵢⱼ, k = 5 · 10⁶ M⁻¹,

### Beispiel 8

### Anlagerung von rPrP-Sonden an bestehende rPrP Aggregate

Zum Aggregationsansatz von Beispiel 7 wurde präaggregiertes rPrP in Konzentrationen von 0,2 bis 20 ng/µl zugegeben. Die rPrP-Aggregate wurden durch Inkubation von rPrP bei einer SDS Konzentration von 0,01% für 1 h bei 25°C erzeugt (c(rPrP) = 100 ng/ml). Der Anlagerungsprozeß wurde durch konsekutive Aufnahme von Auto- und Kreuzkorrelationskurven verfolgt.

Zu beobachten war die Anlagerung von markiertem rPrP an die Aggregate im Zeitbereich von wenigen Minuten. Dieser Prozeß war durch das Auftreten großer Fluoreszenzpeaks in Rohsignal gekennzeichnet. Die Kreuzkorrelationskurven wurden durch eine Komponente mit hoher Diffusionszeit (> 5 ms) dominiert.

### Beispiel 9

### Erkennung von rPrP-Aggregaten durch Kreuzkorrelation zweier Sonden: monoklonaler Antikörper (mAB) + rPrP

Für die spezifische Erkennung von Prion-Protein stehen verschiedene monoklonale Antikörper zur Verfügung, die sich hinsichtlich ihrer Spezifität und Bindungsstärke unterscheiden. Für die Erkennung von pathogenem PrP in Proben von CJD Patienten eignet sich in besonderem Maße ein von B. Oesch (Korth et al., Nature 1997 November 6, 390 (6655): 74-7) entwickelter Antikörper, der die pathogene Form des Prion-Proteins spezifisch bindet. Die Kombination der spezifischer Antikörperbindung mit der Koaggregation von PrP-Sonden bietet eine erhöhte Spezifität gegenüber der einfachen Markierung der pathogenen Aggregate.

Der monoklonale Antikörper IgM 15b3 wurde analog den Prp-Sonden aminofunktional mit Fluoreszenzfarbstoff markiert. Der markierte Antikörper wurde in einer Endkonzentration von 20 nM eingesetzt. Für den gemischten Einsatz von rPrP und Antikörpersonden ist es wünschenswert, daß die Bindungsbedingungen für beide Sonden geeignet sein. Bei einer SDS Konzentration von 0,01% ist sowohl Koaggregation der rPrP-Sonde als auch Antikörperbindung festzustellen. Der Eintritt eines markierten Aggregats in den Fokus erzeugt einen Fluoreszenzpeak. Bei entsprechender Größe dominiert das Peaksignal die Kreuzkorrelationskurve.

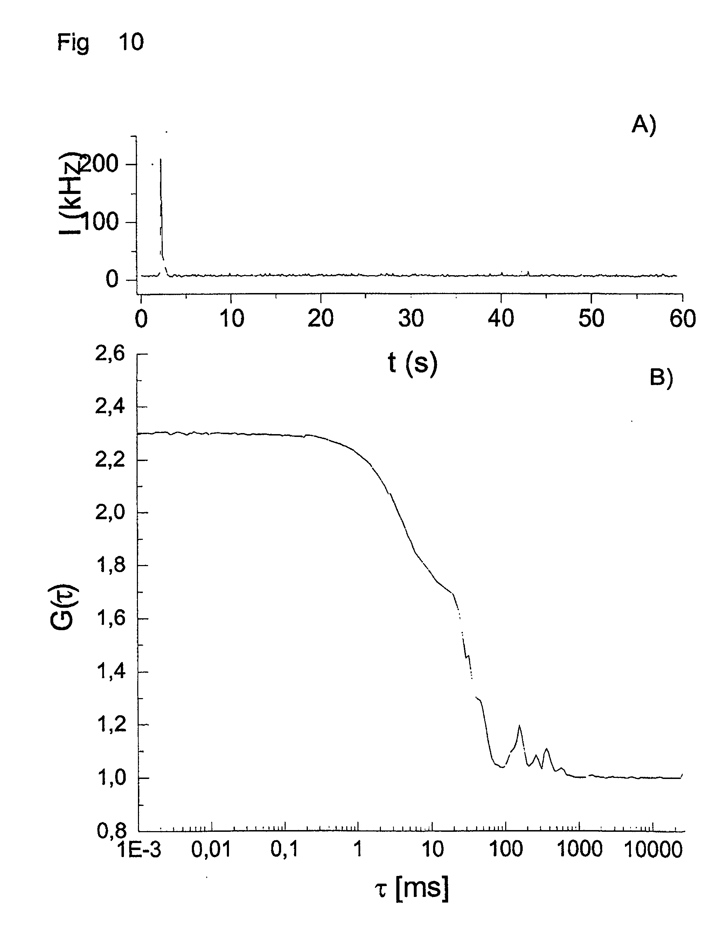

Figur 10 zeigt die Anlagerung von rPrP Sonde und mAB an ein rPrP-Aggregat. Oben: Zeitspur des Kreuzkorrelations-Fluoreszenzsignals. Unten: Zweifarben-Kreuzkorrelationskurve. Der Verlauf der Kurve wird durch das große Aggregat am Beginn der Spur dominiert. Die Konzentrationen sind c(rPrP) = 50 nM, c(rPrP-cy2) = 2 nM, c(mAB-cy5) = 20 nM.

### Beispiel 10

### Peakanalyse der Fluoreszenz-Rohdaten

Durch die Anlagerung vieler Sondenmoleküle an ein Proteintarget entstehen hoch markierte Aggregate (>10 eingelagerte Sonden), die mindestens um den Faktor zwei vor dem Hintergrundsignal der freien Sonde hervortreten. Die Menge der in der Probe vorliegenden Aggregate läßt sich anhand der Zahl der Peaks bestimmen. Bei Meßzeiten von 15 min lassen sich so Aggregate bis zu femtomolaren Konzentrationen detektieren.

Eine Anwendung bildet die Detektion von PrP Aggregaten im Liquor von CJD Patienten. Es wurden drei CJD-negative und drei CJD-positive Liquorproben untersucht. Über die Zahl der Ereignisse mit großen Fluoreszenzbursts konnte zwischen CJD-positiven und CJD-negativen Proben differenziert werden.

Versuchsbedingungen: Cy2 markierte rPrP Sonde wurde bei Anwesenheit von 0,02% SDS zu einer Liquorprobe zugegeben, so daß die Sondenkonzentration 10 nM betrug. Die Messung erfolgte für 15 min bei 22°C.

Datenaufnahme: Die Proben wurden in einem ConfoCor FCS Aufbau vermessen. Die Anregung erfolgte bei einer Wellenlänge von 488 nm (Ar⁺-Laser) mit einer Leistung von 5*104 W/cm². Das emittierte Fluoreszenzlicht wurde durch ein Mikroskopobjektiv (40x /1.2 NA, Zeiss) gesammelt und konfokal auf eine Avalanche-Photodiode abgebildet. Diese wirkt als Photonenzähler, der für jedes Photon einen Signalpuls liefert. Die Signalpulse wurden über einen Signalteiler parallel auf einer Harwarekorrelatorkarte ausgewertet und auf einer Scalerkarte mit einer Kanalbreite von 250 µs aufgezeichnet. Die Gesamtmeßzeit betrug 15 min, was einer Zahl von 3.6 Millionen Kanälen entspricht.

Datenauswertung: Die Auswertung erfolgte nach der Zahl und Höhe der Fluroeszenzpeaks. Eine Verteilung der Anzahl der Photonen pro Kanal ist in einem Histogramm dargestellt.

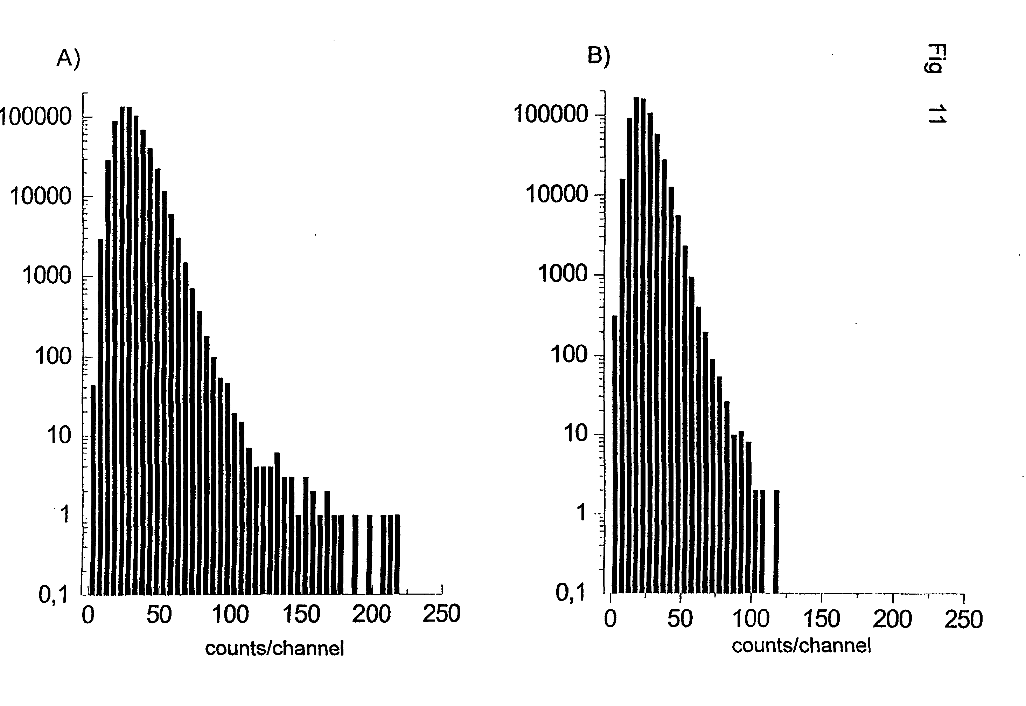

Figur 11 zeigt die Häufigkeitsverteilung der Fluoreszenzphotonen pro Meßkanal bei einer Kanalbreite von 250 µs und Konzentration der Sonde rPrP-Cy2 = 20 nM; links: CJD-positive Liquorprobe, rechts: CJD-negative Liquorprobe.

Dabei gibt die Photonenzahl die Höhe der Peaks, während die Kanalzahl ein Maß für das Integral über die Fläche aller Peaks ist. Man erkennt in den CJD positiven Proben einen höheren Anteil großer Fluoreszenzpeaks. Durch Setzen eines Schwellenwertes kann man klar zwischen CJD positiven und negativen Proben differenzieren.

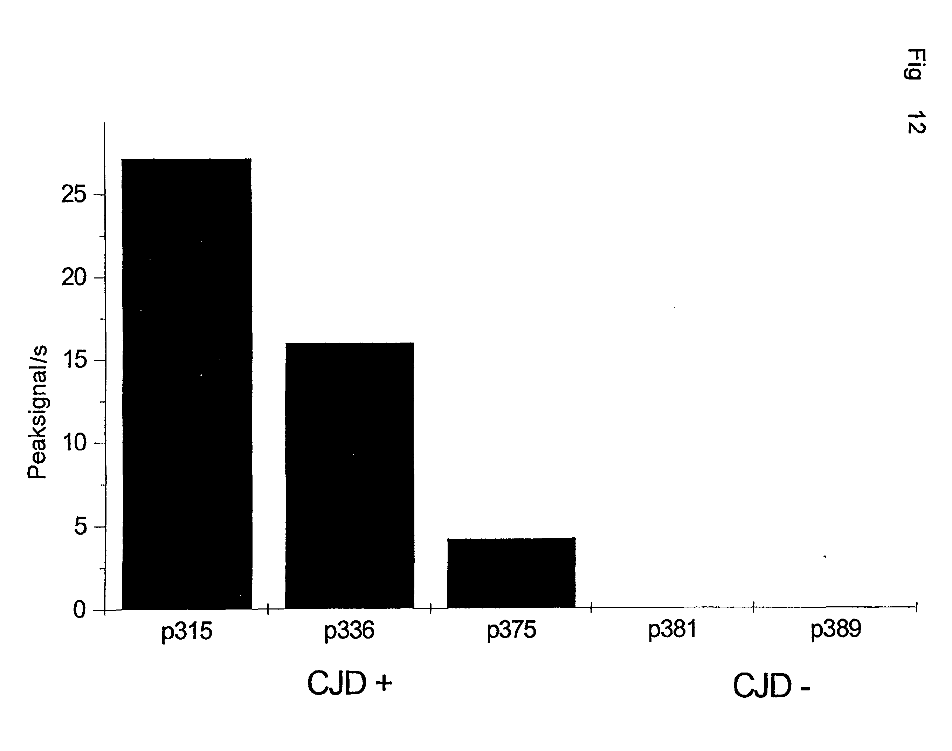

Figur 12 zeigt die Häufigkeit von Fluoreszenzphotonen aus Kanälen mit mehr als 150 counts/channel in CJD-positiven (CJD+)./CJD-negativen (CJD-) Liquorproben bei einer Kanalbreite von 250 µs und einer Konzentration der Sonde rPrP-Cy2 von 20 nM. p315 etc. ist eine interne Identifizierungsnummer der Patienten.

Im zweifarbigen Aufbau läßt sich durch eine Multiplikation des roten und des grünen Signals eine Koinzidenzanalyse durchführen, um so Peaks zu erkennen, die mit Sonden beider Farben markiert sind. Durch eine verfeinerte Peak-Burst Analyse ließe sich die Trennung großer Peaks vom Signal der Sonde weiter verbessern.

### Beispiel 11

### Congo-Rot als Modell für das Screeningverfahren

Congo-Rot ist bekannt für seinen inhibierenden Effekt auf die Fibrillogenese.

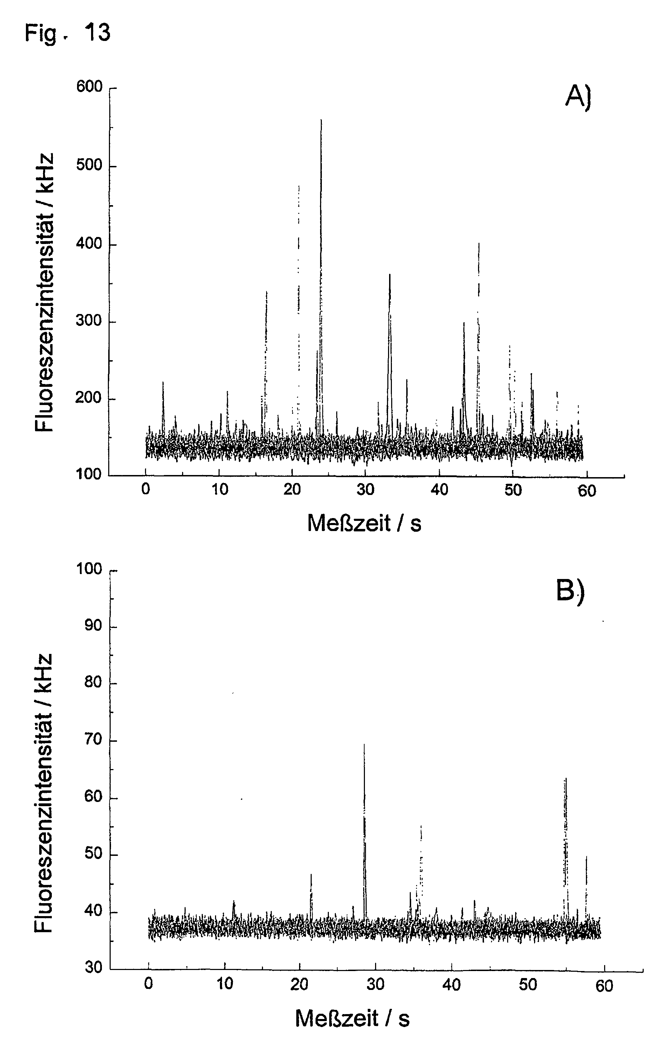

Die Original-Messung (jeweils 20 Messungen mit einer Minute Meßzeit) im Liquor eines an der Alzheimerschen Demenz erkrankten Patienten ist in der Figur 13 dargestellt.

Figur 13 zeigt den Einfluß von Congo-Rot auf die Anlagerung der spezifischen Sonde Aß-42-CY2 an synthetische Aßl-42-Aggregate. Im FCS kann bei Zugabe von 1 mM Congo-Rot eine Absinken der Signalintensität der homologen Anlagerung auf 20% beobachtet werden.

Durch Zugabe von 1 mM Congo-Rot (unten) ist ein deutliches Absinken der detektierbaren Peaks gegenüber dem Kontrollansatz (oben) zu beobachten. Aufgrund der Absorption von Congo-Rot im Bereich der Anregungswellenlänge ist eine unterschiedliche Skalierung der Messung für Proben mit und ohne Congo-Rot vorgenommen worden.

Dies zeigt, daß Substanzen, die die Fibrillogenese hemmen, im FCS identifiziert werden können.

## Patentansprüche

1. Verfahren zur diagnostischen Erfassung von Erkrankungen, die mit Proteinablagerungen verbunden sind (pathologische Proteinablagerungen), durch Messung einer Assoziation von Teilstrukturen der pathologischen Proteinablagerungen, pathologische Proteinablagerungen bildenden Strukturen, pathologische Proteinablagerungen entsprechenden Strukturen und/oder pathologischen Proteinablagerungen als Sonde,
an Teilstrukturen der pathologischen Proteinablagerung, pathologische Proteinablagerungen bildende Strukturen, pathologischen Proteinablagerungen entsprechende Strukturen und/oder pathologische Proteinablagerungen als Target,
**dadurch gekennzeichnet, daß** das Target in flüssiger Phase nachgewiesen wird, wobei im Falle einer Erfassung von Alzheimer-Erkrankungen die flüssige Phase aus Körperflüssigkeiten gewonnen wurde oder eine Körperflüssigkeit ist,
mit der Maßgabe, daß die Assoziation der Sonde an das Target gemessen wird, bevor eine Selbstaggregation der Sonde überwiegt.

2. Verfahren nach Anspruch 1, wobei die Teilstrukturen der pathologischen Proteinablagerungen, die pathologische Proteinablagerungen bildenden Strukturen, die pathologischen Proteinablagerungen entsprechenden Strukturen und/oder die pathologischen Proteinablagerungen mindestens eine detektierbare Eigenschaft aufweisen.

3. Verfahren nach Anspruch 1 und/oder 2, wobei die Teilstrukturen der pathologischen Proteinablagerungen mit mindestens einer detektierbaren Eigenschaft monomere oder oligomere Einheiten pathologischer Proteinablagerungen sind oder die Teilstrukturen homolog zu monomeren oder oligomeren Einheiten pathologischer Proteinablagerungen sind.

4. Verfahren nach einem der Ansprüche 1 bis 3, wobei Sonde und Target aus dem gleichen oder einem anderen Typ pathologischer Proteinablagerungen stammen.

5. Verfahren nach einem der Ansprüche 1 bis 4, wobei die Teilstrukturen durch Behandlung pathologischer Proteinablagerungen mit physikalischen Methoden, wie Ultraschallbehandlung, Einwirkung von Temperaturänderungen, oder mit chemischen Methoden, wie Behandlung mit Lösungen unterschiedlicher Ionenstärke, Behandlung mit Lösungen chaotroper Ionen, Behandlung mit Detergenzien und/oder mit Enzymen, insbesondere Proteasen, erhältlich sind.

6. Verfahren nach einem der Ansprüche 1 bis 5, wobei die mindestens eine detektierbare Eigenschaft entweder bereits in der Teilstruktur vorhanden ist oder nachträglich eingeführt wird.

7. Verfahren nach einem der Ansprüche 1 bis 6, wobei zur Erhöhung der Spezifität oder Selektivität der diagnostischen Erfassung andere mit den Targets in Wechselwirkung tretende Substanzen eingesetzt werden.

8. Verfahren nach einem der Ansprüche 2 bis 7, wobei die mindestens eine detektierbare Eigenschaft Größe oder Ausdehnung. Molmasse, Struktur, Zirkular-Dichroismus, optische Eigenschaft wie Lumineszenz, insbesondere Fluoreszenz, Absorption, Radioaktivität ist.

9. Verfahren nach einem der Ansprüche 2 bis 8, wobei die Messung der mindestens einen detektierbaren Eigenschaft mittels physikalischer Methoden, insbesondere spektroskopischer Methoden, erfolgt.

10. Verfahren nach einem der Ansprüche 2 bis 9, wobei die Messung der mindestens einen detektierbaren Eigenschaft mittels fluorimetrischer Methoden wie konfokaler Fluoreszenzspektroskopie, Fluoreszenz-Korrelations-Spektroskopie (FCS), FCS in Kombination mit Kreuzkorrelation jeweils mit entsprechenden Auswertungsverfahren erfolgt.

11. Verfahren nach einem der Ansprüche 1 bis 10, wobei die pathologischen Proteinablagerungen amyloide Plaques und/oder neurofibrilläre Filamente (NFT) sind, die aus dem neuronalen System stammen und mit neurodegenerativen Erkrankungen, wie Morbus Alzheimer, Transmissible spongioformer Enzephalopathie, Chorea Huntington, Morbus Parkinson, Vererbbare Cerebrale Amyloide Angiophathi einhergehen.

12. Verfahren nach einem der Ansprüche 1 bis 10, wobei die pathologischen Proteinablagerungen insbesondere amyloide Plaques sind, die aus anderen Organen als dem neuronalen System stammen und mit Erkrankungen wie insbesondere Primärer Aystemischer Amyloidose (AL-Amyloidose), Reaktiver Aystemischer Amyloidose (AA-Amyloidose), Familiärer Amyloider Polyneuropathie, Typ II Diabetes, Injektions-Lokalisierter Amyloidose, Medullaria-Carcinom der Schilddrüse, Beta-2-Microglobulin Amyloidose, Vererbter Nichtneuropatischer Amyloidose, Finnischer Vererbter Systemischer Amyloidose einhergehen.

13. Verfahren nach einem der Ansprüche 1 bis 12, wobei die Sonden rekombinante Proteine, Proteinfragmente oder Peptide sind, die Sequenzhomologien zu amyloiden Plaques und/oder neurofibrillären Filamenten aufweisen.

14. Verfahren nach einem der Ansprüche 1 bis 13, wobei aus Körperflüssigkeiten wie Liquor cerebrospinalis, Blut, Lymphe, Urin, oder Sekreten wie Sputum oder Gewebe, Proben entnommen und zur diagnostischen Erfassung etwaiger Erkrankungen mit Sonden in Kontakt gebracht, inkubiert und deren Assoziation an Targets gemessen werden.

15. Verfahren nach einem der Ansprüche 2 bis 14, wobei aus den detektierbaren Eigenschaften Parameter wie Translationsdiffusionsgeschwindigkeiten, Rotationsdiffusionsgeschwindigkeiten, Lebensdauern angeregter Zustände, Polarisation von Strahlung, Energietransfer, Quantenausbeute, Partikelzahl oder Konzentration, Intensitätsdifferenzen ermittelt werden.

## Claims

1. A method for the diagnostic detection of diseases associated with protein depositions (pathological protein depositions) by measuring an association of substructures of the pathological protein depositions, structures forming pathological protein depositions, structures corresponding to pathological protein depositions and/or pathological protein depositions as a probe;
to substructures of the pathological protein depositions, structures forming pathological protein depositions, structures corresponding to pathological protein depositions and/or pathological protein depositions as targets;
**characterized in that** the target is detected in liquid phase wherein, in the case of detecting Alzheimer's disease, the liquid phase is obtained from body fluids or is itself a body fluid;
with the proviso that the association of the probe to the target is measured before self-aggregation of the probe predominates.

2. The method according to claim 1, wherein said substructures of the pathological protein depositions, structures forming pathological protein depositions, structures corresponding to pathological protein depositions and/or pathological protein depositions have at least one detectable property.

3. The method according to claim 1 and/or 2, wherein said substructures of the pathological protein depositions having at least one detectable property are monomeric or oligomeric units of pathological protein depositions, or the substructures are homologous with monomeric or oligomeric units of pathological protein depositions.

4. The method according to any of claims 1 to 3, wherein said probe and said target are derived from the same or from different types of pathological protein depositions.

5. The method according to any of claims 1 to 4, wherein said substructures can be obtained by treating pathological protein depositions with physical means, such as ultrasonication, the action of temperature changes, or with chemical means, such as treatment with solutions of different ionic strengths, treatment with solutions of chaotropic ions, treatment with detergents and/or enzymes, especially proteases.

6. The method according to any of claims 1 to 5, wherein said at least one detectable property is either intrinsic to the substructure, or it is introduced later.

7. The method according to any of claims 1 to 6, wherein other substances which will interact with the targets are used to increase the specificity or selectivity of the diagnostic detection.

8. The method according to any of claims 2 to 7, wherein said at least one detectable property is size or dimension, molecular weight, structure, circular dichroism, optical properties such as luminescence, especially fluorescence, absorption, radioactivity.

9. The method according to any of claims 2 to 8, wherein the measurement of said at least one detectable property is performed by physical methods, especially spectroscopic methods.

10. The method according to any of claims 2 to 9, wherein the measurement of said at least one detectable property is performed by fluorimetric methods, such as confocal fluorescence spectroscopy, fluorescence correlation spectroscopy (FCS), FCS in combination with cross-correlation, with their respective appropriate evaluation methods.

11. The method according to any of claims 1 to 10, wherein said pathological protein depositions are amyloid plaques and/or neurofibrillar filaments (NFT) which are derived from the neuronal system and are associated with neurodegenerative diseases, such as Alzheimer's disease, transmissible spongiform encephalopathy, Huntington's chorea, Parkinson's disease, hereditary cerebral amyloid angiopathy.

12. The method according to any of claims 1 to 10, wherein said pathological protein depositions are, in particular, amyloid plaques derived from organs other than the neuronal system and are associated with diseases such as, in particular, primary systemic amyloidosis (AL amyloidosis), reactive systemic amyloidosis (AA amyloidosis), familial amyloid polyneuropathy, type II diabetes, injection-localized amyloidosis, medullary carcinoma of the thyroid gland, beta-2 microglobulin amyloidosis, hereditary non-neuropathic amyloidosis, Finnish hereditary systemic amyloidosis.

13. The method according to any of claims 1 to 12, wherein said probes are recombinant proteins, protein fragments or peptides having sequence homologies with amyloid plaques and/or neurofibrillar filaments.

14. The method according to any of claims 1 to 13, wherein samples are taken from body fluids, such as cerebrospinal fluid, blood, lymph, urine, or secretions such as sputum, or tissue and contacted with probes for the diagnostic detection of any diseases, incubated, and the association of the probes to targets is measured.

15. The method according to any of claims 2 to 14, wherein parameters such as translational diffusion rates, rotational diffusion rates, lifetimes of excited states, polarization of radiation, energy transfer, quantum yield, number or concentration of particles, intensity differences are established from said detectable properties.

## Revendications

1. Procédé de détection diagnostique de pathologies liées à des dépôts protéiques (dépôts protéiques pathologiques) par mesure d'une association de structures partielles des dépôts protéiques pathologiques, de structures formant des dépôts protéiques pathologiques, de structures correspondant à des dépôts protéiques pathologiques et/ou de dépôts protéiques pathologiques en tant que sonde, à des structures partielles du dépôt protéique pathologique, des structures formant des dépôts protéiques pathologiques, des structures correspondant à des dépôts protéiques pathologiques et/ou des dépôts protéiques pathologiques en tant que cible, **caractérisé en ce que** la cible est détectée en phase liquide, la phase liquide étant obtenue, dans le cas de la détection des pathologies d'Alzheimer, à partir de liquides corporels ou étant un liquide corporel, pourvu que l'association de la sonde à la cible soit mesurée avant qu'une auto-agrégation de la sonde ne prédomine.

2. Procédé selon la revendication 1 dans lequel les structures partielles des dépôts protéiques pathologiques, les structures formant des dépôts protéiques pathologiques, les structures correspondant à des dépôts protéiques pathologiques et/ou les dépôts protéiques pathologiques présentent au moins une propriété détectable.

3. Procédé selon la revendication 1 et/ou la revendication 2 dans lequel les structures partielles des dépôts protéiques pathologiques ayant au moins une propriété détectable sont des unités monomères ou oligomères de dépôts protéiques pathologiques ou bien dans lequel les structures partielles sont homologues à des unités monomères ou oligomères de dépôts protéiques pathologiques.

4. Procédé selon l'une des revendications 1 à 3 dans lequel la sonde et la cible proviennent du même type ou de différents types de dépôts protéiques pathologiques.

5. Procédé selon l'une des revendications 1 à 4 dans lequel les structures partielles peuvent être obtenues par traitement de dépôts protéiques pathologiques selon des méthodes physiques, comme le traitement aux ultrasons, l'action de modifications de la température ou selon des méthodes chimiques comme le traitement avec des solutions de différentes forces ioniques, le traitement avec des solutions d'ions chaotropes, le traitement avec des détergents et/ou des enzymes, en particulier des protéases.

6. Procédé selon l'une des revendications 1 à 5 dans lequel la au moins une propriété détectable soit est déjà présente dans la structure partielle soit est introduite postérieurement

7. Procédé selon l'une des revendications 1 à 6 dans lequel on utilise d'autres substances qui interagissent avec les cibles pour augmenter la spécificité ou la sélectivité de la détection diagnostique.

8. Procédé selon l'une des revendications 2 à 7 dans lequel la au moins une propriété détectable est la taille ou l'étendue, la masse molaire, la structure, le dichroïsme circulaire, une propriété optique telle que la luminescence, en particulier la fluorescence, l'absorption, la radioactivité.

9. Procédé selon l'une des revendications 2 à 8 dans lequel la mesure de la au moins une propriété détectable est réalisée au moyen de méthodes physiques, en particulier de méthodes spectroscopiques.

10. Procédé selon l'une des revendications 2 à 9 dans lequel la mesure de la au moins une propriété détectable est réalisée au moyen de méthodes fluorimétriques comme la spectroscopie de fluorescence confocale, la spectroscopie à corrélation de fluorescence (FCS), la FCS en combinaison avec la corrélation croisée, chacune avec les méthodes d'analyse correspondantes.

11. Procédé selon l'une des revendications 1 à 10 dans lequel les dépôts protéiques pathologiques sont des plaques amyloïdes et/ou des filaments neurofibrillaires (NFT) provenant du système neuronal et qui vont de paire avec des pathologies neurodégénératives comme la maladie d'Alzheimer, l'encéphalopathie spongiforme transmissible, la chorée de Huntington, la maladie de Parkinson, l'angiopathie amyloïde cérébrale transmissible.

12. Procédé selon l'une des revendications 1 à 10 dans lequel les dépôts protéiques pathologiques sont en particulier des plaques amyloïdes provenant d'organes autres que le système neuronal et vont de paire avec certaines pathologies comme, en particulier, l'amyloïdose immunoglobulinique (amyloïdose AL), l'amyloïdose inflammatoire (amyloïdose AA), la neuropathie amyloïde familiale, le diabète de type II, l'amyloïdose localisée à la zone d'injection, le carcinome médullaire de la glande tyroïde, l'amyloïdose microglobulinique bêta 2, l'amyloïdose non neuropathologique héréditaire, l'amyloïdose systémique héréditaire type finnoise.

13. Procédé selon l'une des revendications 1 à 12 dans lequel les sondes sont des protéines recombinantes, des fragments protéiques ou des peptides présentant des homologies de séquence avec des plaques amyloïdes et/ou des filaments neurofibrillaires.

14. Procédé selon l'une des revendications 1 à 13 dans lequel on prélève des échantillons dans les liquides corporels comme le liquide cérébro-spinal, le sang, la lymphe, l'urine ou dans les sécrétions comme les crachats ou les tissus et dans lequel on les met en contact avec des sondes aux fins de détection diagnostique d'éventuelles pathologies, on les incube et on mesure leur association sur des cibles.

15. Procédé selon l'une des revendications 2 à 14, dans lequel on détermine, à partir des propriétés détectables, un paramètre comme des vitesses de diffusion par translation, des vitesses de diffusion par rotation, des états de durée de vie stimulée, une polarisation de rayonnement, un transfert d'énergie, un rendement quantique, un nombre ou une concentration de particules, des différences d'intensité.
